# EUROPEAN PATENT SPECIFICATION

(11) **EP 3 033 130 B1**
(45) Date of publication and mention of the grant of the patent: **11.10.2017**
(21) Application number: 14777801.3
(22) Date of filing: 29.07.2014
(51) Int. Cl.: A61M 16/06

(54) **DETECTING THE FIT OF A PATIENT INTERFACE DEVICE**
ERKENNUNG DES SITZES EINER PATIENTENSCHNITTSTELLENVORRICHTUNG
DÉTECTION DE L'AJUSTEMENT D'UN DISPOSITIF D'INTERFACE PATIENT

(30) Priority: 12.08.2013 US 201361864878 P
(43) Date of publication of application: 22.06.2016
(73) Proprietor: Koninklijke Philips N.V., 5656 AE Eindhoven (NL)
(72) Inventor: GRASHOW, Jonathan Sayer, 5656 AE Eindhoven (NL)
(74) Representative: Schudelaro, Antonius Adrianus Petrus
(86) International application number: PCT/IB2014/063491
(87) International publication number: WO 2015/022595

(56) References cited:
- US-A1- 2013 032 142
- US-A1- 2013 180 523
- US-B1- 7 204 250

## Description

### BACKGROUND OF THE INVENTION

### 1. Field of the Invention

The present invention pertains to patient interface devices structured to deliver a flow of breathing gas to a patient, and, in particular, to various methods and apparatus for automatically detecting the fit of a patient interface device using Projected Capacitive Technology (PCT).

### 2. Description of the Related Art

There are numerous situations where it is necessary or desirable to deliver a flow of breathing gas non-invasively to the airway of a patient, i.e., without intubating the patient or surgically inserting a tracheal tube into the patient's esophagus. For example, it is known to ventilate a patient using a technique known as non-invasive ventilation. It is also known to deliver positive airway pressure (PAP) therapy to treat certain medical disorders, the most notable of which is obstructive sleep apnea (OSA). Known PAP therapies include continuous positive airway pressure (CPAP), in which a constant positive pressure is provided to the airway of the patient in order to splint open the patient's airway, and variable airway pressure, in which the pressure provided to the airway of the patient is varied with the patient's respiratory cycle and/or based on the monitored condition of the patient. Such therapies are typically provided to the patient at night while the patient is sleeping.

Non-invasive ventilation and pressure support therapies as just described involve the placement of a patient interface device including a mask component having a soft, flexible sealing cushion on the face of a patient. The mask component may be, without limitation, a nasal mask that covers the patient's nose, a nasal/oral mask that covers the patient's nose and mouth, a nasal cushion that is positioned beneath and engages the patient's nose, or a full face mask that covers the patient's face. Such patient interface devices may also employ other patient contacting components, such as forehead supports, cheek pads and chin pads. The sealing cushion typically has a support portion coupled to a sealing flap portion, which may integrated together as a single part or that may be separate components that when combined together in the final assembly provide the sealing and support functions. The patient interface device is connected to a gas delivery tube or conduit and interfaces the ventilator or pressure support device with the airway of the patient, so that a flow of breathing gas can be delivered from the pressure/flow generating device to the airway of the patient. It is known to maintain such devices on the face of a wearer by a headgear having one or more straps adapted to fit over/around the patient's head.

A requisite of such patient interface devices used in non-invasive ventilation and pressure support therapies is that they provide an effective seal against the user's face to prevent leakage of the gas being supplied, while also providing a comfortable user/seal interface. This problem is significant because such patient interface devices are typically worn for an extended period of time. For example, in the case of patient interface devices used to provide pressure support therapies to treat medical disorders as described above, the device is worn for several hours in bed. Such extended use can create several discomfort problems for the user, such as the formation of red marks on the user's face, skin irritation, and/or heat and moisture discomfort. These discomfort problems can lead to reduced therapy compliance by patients as they may wish to avoid wearing an uncomfortable mask.

US 2013/180523 A1 discloses a respiratory therapy system and a method according to the preambles of the independent claims. This known respirator has one or more electrodes of e.g. conductive elastomer disposed on the surface of a face sealing member opposite to the surface which seals against the user's face. In use, the integrity of the seal formed between the sealing member and the user's face is monitored by monitoring the electrical capacitance across that member between the electrode or electrodes and the user's face.

US 7 204 250 B1 discloses a breathing mask for use in monitoring a patient. The mask can have sensors on the body of the mask and on the associated straps or caps. The mask can be used to monitor breathing problems or to monitor a patient during anesthesia. The data acquired from the mask sensors and related sensors can be stored or fed into a computer to analyze the patient's condition and provide feedback information.

US 2013/032142 A1 discloses a system, method, and apparatus for air delivery. A formable mask has an oxygen supply port and exhaled air removal components for the delivery of oxygen enriched air to a user. The formable mask includes formable components and materials to increase comfort and minimize chafing of a wearer.

### SUMMARY OF THE INVENTION

According to an aspect of the invention, a respiratory therapy system and a method of detecting a fit of a patient interface device according to the independent claims are provided.

In one embodiment, a method of detecting a fit of a patient interface device is provided. The method includes determining a plurality of capacitance values when the patient interface device is donned by a user. The patient interface device has a contacting element having a user contacting surface, wherein each of the capacitance values is associated with one of a plurality of locations along at least a portion of the contacting surface. Each of the locations has a number of electrode elements associated therewith that are used to determine the capacitance values, classify each of the capacitance values into one of a plurality of predetermined categories, and use the classified capacitance values to identify a number of predetermined fit conditions along the contacting element.

In another embodiment, a respiratory therapy system is provided that includes a patient interface device having a contacting element having a user contacting surface, wherein each of a plurality of locations along at least a portion of the contacting surface has a number of electrode elements associated therewith, a sensing circuit coupled to the electrode elements, and a processing apparatus in communication with the sensing circuit, the sensing circuit and the processing apparatus being structured to: (i) determine a plurality of capacitance values using the electrode elements when the patient interface device is donned by a user, wherein each of the capacitance values is associated with one the locations, (ii) classify each of the capacitance values into one of a plurality of predetermined categories, and (iii) use the classified capacitance values to identify a number of predetermined fit conditions along the contacting element.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

FIGS. 1 and 2 are front and side schematic views, respectively, of a system adapted to provide a regimen of respiratory therapy to a patient according to one exemplary embodiment;
FIG. 3 is a schematic diagram of a pressure generating device forming part of the system of FIGS. 1 and 2 according to one particular, non-limiting exemplary embodiment;
FIG. 4 is a schematic diagram showing two alternative implementations of a mask component forming part of the system of FIGS. 1 and 2 according to one particular, non-limiting exemplary embodiment;
FIG. 5 is a flowchart illustrating a method of detecting the degree of contact between the user's face and various regions of the sealing surface of the mask component of FIGS. 1-4 according to one particular, non-limiting exemplary embodiment;
FIG. 6 is a schematic diagram showing a visual map indicating regions of potential leak, optimum fit and over-tightening according to one particular, non-limiting exemplary embodiment;
FIG. 7 is a front schematic diagram showing an alternative implementation of a patient interface device that may form part of the system of FIGS. 1 and 2 according to an alternative exemplary embodiment;
FIG. 8 is a schematic diagram showing a visual map indicating regions of potential leak, optimum fit and over-tightening according to another particular, non-limiting exemplary embodiment;
FIGS. 9 and 10 are front and side schematic views, respectively, of a system adapted to provide a regimen of respiratory therapy to a patient according to an alternative exemplary embodiment;
FIG. 11 is a schematic diagram of a pressure generating device forming part of the system of FIGS. 9 and 10 according to one particular, non-limiting exemplary embodiment;
FIG. 12 is a schematic diagram of a local electronic device forming part of the patient interface device of the system of FIGS. 9 and 10 according to one particular, non-limiting exemplary embodiment; and
FIG. 13 is a front schematic view of a system adapted to provide a regimen of respiratory therapy to a patient according to another alternative exemplary embodiment.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

As used herein, the word "outline" means a line enclosing or indicating the shape of an object or a portion of an object, such as, without limitation, a surface of an object. Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

Projected Capacitive Technology (PCT) has recently become widely used in products that employ touch screen interfaces (e.g., a number of different commercially available smartphones such as the iPhone®). In general, PCT falls into one of two implementation categories: (i) self-capacitance technology, and (ii) mutual capacitance technology. Both self-capacitance technology and mutual capacitance technology employ an array of electrodes embedded beneath a dielectric material (e.g., a glass screen). In self-capacitance technology, each electrode functions as one half of a capacitor, and when a finger or other conductor comes into relatively close proximity (typically < 20 mm) with the charged electrode, a current is driven through the finger (which effectively becomes the other "plate" in the capacitor) and the capacitance associated with that electrode changes (i.e., as compared to a baseline value). Electronics are used to measure the current on each electrode to ground and/or the change in capacitance (ΔC) associated with that electrode.

In mutual capacitance technology, the capacitance is measured between a number neighboring charged electrodes (termed a "mutual capacitance"). When a finger or other conductor comes into close proximity (typically < 2 mm) with the neighboring charged electrodes, the mutual capacitance between the neighboring electrodes is changed (i.e., as compared to a baseline value) due to disruption of the electrical field. Electronics are used to measure the change in mutual capacitance (ΔMC) associated with the neighboring electrodes. In both self-capacitance and mutual capacitance technology, the measured capacitance information (ΔC and ΔMC) is used to track the position and movement of the finger or other conductor relative to the electrode array.

As described in detail herein, the principles of projected capacitance (e.g., self capacitance or mutual capacitance) are used to detect contact between the face of a patient and the sealing surface of a contact element (such as a cushion member) of a patient interface device to enable automated determination of predetermined fit conditions, such as potential leak locations, over-tightened regions and/or regions of optimum fit, on the patient interface device. More specifically, and as described in more detail herein in connection with the various exemplary embodiments of the present invention, an array of electrodes is provided on or embedded within a portion of a patient interface device (such as, without limitation, a mask support portion or sealing flap portion of a cushion member of the patient interface device or another contacting element of the patient interface device, such as a forehead pad or cheek pad), and ΔC or ΔMC measurements are made (after the patient interface device is donned by the patient).

The magnitude of such measurements are then used to detect the degree of contact between the face and various regions of the sealing surface of the patient interface device to identify locations of predetermined fit conditions, such as potential leak, over-tightening and/or optimum fit. As described elsewhere herein, in the exemplary embodiments, the ΔC or ΔMC measurements are used to create a data map which identifies such regions along the outline of the sealing surface of the patient interface device, which data map may be used to generate a visual map for visualizing those regions. Other potential uses of the data map information are also described herein.

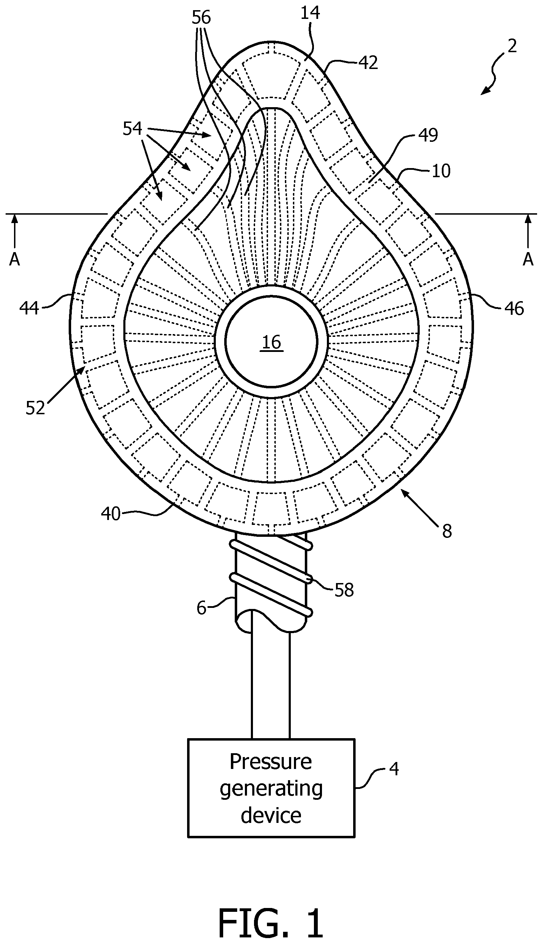

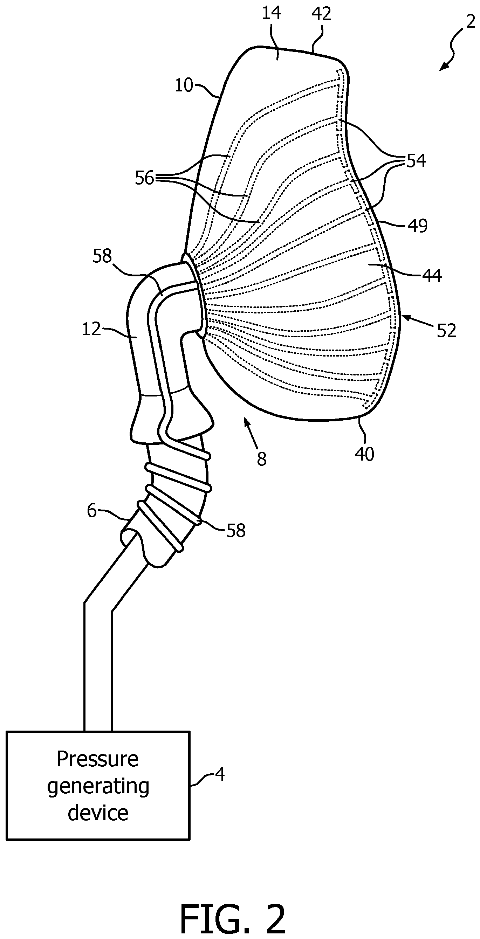

FIGS. 1 and 2 are front and side schematic views, respectively, of a system 2 adapted to provide a regimen of respiratory therapy to a patient according to one exemplary embodiment. For illustrative purposes, and as described in detail herein, in the exemplary embodiment of FIGS. 1 and 2, self capacitance technology is employed to enable the automated determination of potential leak, over-tightened and/or optimum fit regions of a patient interface device. It will be understood, however, that that is meant to be exemplary only and that other PCT implementations, such as mutual capacitance technology, may also be employed to implement the principles of the present invention.

As seen in FIGS. 1 and 2, system 2 includes a pressure generating device 4, a delivery conduit 6, and a patient interface device 8 including a mask component 10 and an elbow conduit 12. Pressure generating device 4 is structured to generate a flow of breathing gas and may include, without limitation, ventilators, constant pressure support devices (such as a continuous positive airway pressure device, or CPAP device), variable pressure devices (e.g., BiPAP®, Bi-Flex®, or C-Flex™ devices manufactured and distributed by Philips Respironics of Murrysville, Pennsylvania), and auto-titration pressure support devices. Delivery conduit 6 is structured to communicate the flow of breathing gas from pressure generating device 4 to patient interface device 8.

In the illustrated embodiment, mask component 10 comprises a nasal mask structured to cover the nose of the patient. However, other types of mask components, such as, without limitation, a nasal/oral mask that covers the patient's nose and mouth, a nasal cushion that is positioned beneath and engages the patient's nose, or a full face mask that covers the patient's face, which facilitate the delivery of the flow of breathing gas to, and the removal of a flow of exhalation gas from, the airway of a patient may be used while remaining within the scope of the present invention. In the embodiment shown in FIGS. 1 and 2, mask component 10 includes a cushion member 14 having an opening 16 to which elbow conduit 12 is coupled to allow the flow of breathing gas from pressure generating device 4 to be communicated to an interior space defined by cushion member 14, and then, to the airway of a patient. In an alternative embodiment (not shown), cushion member may be coupled to a frame member made of a rigid or semi-rigid material, such as, without limitation, an injection molded thermoplastic or silicone, for providing additional structural support. As is known, such a frame member may include elements for attachment of a headgear component and/or a forehead, cheek and/or chin support component to mask component 10 as part of patient interface device 8.

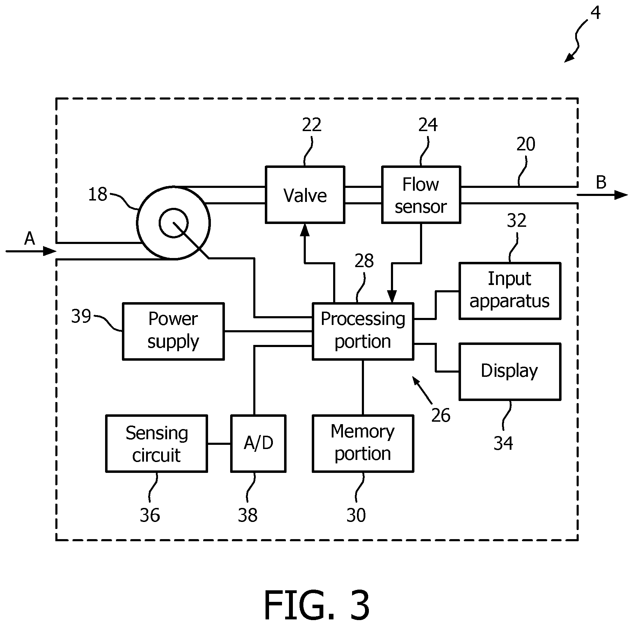

FIG. 3 is a schematic diagram of pressure generating device 4 according to one particular, non-limiting exemplary embodiment. Referring to FIG. 3, pressure generating device 4 includes a gas flow generator 18, such as a blower used in a conventional CPAP or bi-level pressure support device, which receives breathing gas, generally indicated by arrow A, from any suitable source, e.g., a pressurized tank of oxygen or air, the ambient atmosphere, or a combination thereof. Gas flow generator 18 generates a flow of breathing gas, such as air, oxygen, or a mixture thereof, for delivery to an airway of a patient at relatively higher and lower pressures, i.e., generally equal to or above ambient atmospheric pressure. In the exemplary embodiment, gas flow generator 18 is capable of providing a flow of breathing gas ranging in pressure from 3-30 cmH₂O. The pressurized flow of breathing gas, generally indicated by arrow B from gas flow generator 18, is delivered via a delivery tube 20 to patient interface device 8 through delivery conduit 6 as described herein.

In the illustrated embodiment, pressure generating device 4 includes a pressure controller in the form of a valve 22 provided in delivery tube 20. Valve 22 controls the pressure of the flow of breathing gas from flow generator 18 that is delivered to the patient. For present purposes, flow generator 18 and valve 22 are collectively referred to as a pressure generating system because they act in concert to control the pressure and/or flow of gas delivered to the patient. However, it should be apparent that other techniques for controlling the pressure of the gas delivered to the patient, such as varying the blower speed of flow generator 18, either alone or in combination with a pressure control valve, are contemplated by the present invention. Thus, valve 22 is optional depending on the technique used to control the pressure of the flow of breathing gas delivered to the patient. If valve 22 is eliminated, the pressure generating system corresponds to flow generator 18 alone, and the pressure of gas in the patient circuit is controlled, for example, by controlling the motor speed of flow generator 18.

Pressure generating device 4 further includes a flow sensor 24 that measures the flow of the breathing gas within delivery tube 20. In the particular embodiment shown in FIG. 3, flow sensor 24 is interposed in line with delivery tube 20, most preferably downstream of valve 22. Flow sensor 24 generates a flow signal, Q_{measured}, that is provided to a processing apparatus 26 and is used by processing apparatus 26 to determine the flow of gas at the patient (Qₚₐₜᵢₑₙₜ).

Techniques for calculating Qₚₐₜᵢₑₙₜ based on Q_{measured} are well known, and take into consideration the pressure drop of the patient circuit, known leaks from the system, i.e., the intentional exhausting of gas from the circuit, and unknown leaks from the system, such as leaks at patient interface device 8. The present invention contemplates using any known or hereafter developed technique for calculating leak flow Qₗₑₐₖ, and using this determination in calculating Qₚₐₜᵢₑₙₜ based on Q_{measured}. Examples of such techniques are taught by U.S. Patent Nos. 5,148,802; 5,313,937; 5,433,193; 5,632,269; 5,803,065; 6,029,664; 6,539,940; 6,626,175; and 7,011,091.

Of course, other techniques for measuring the respiratory flow of the patient are contemplated by the present invention, such as, without limitation, measuring the flow directly at the patient or at other locations along delivery tube 20, measuring patient flow based on the operation of flow generator 18, and measuring patient flow using a flow sensor upstream of valve 22.

Processing apparatus 26 includes a processing portion 28, that may be, for example, a microprocessor, a microcontroller or some other suitable processing device, and a memory portion 30, that may be internal to the processing portion 28 or operatively coupled to processing portion 28 and that provides a storage medium for data and software executable by processing portion 28 for controlling the operation of pressure generating device 4 as described in greater detail herein.

An input apparatus 32 is provided for setting various parameters used by pressure generating device 4, and a display 34 is provided for displaying and outputting information and data to a user, such as the patient or a clinician or caregiver, as described elsewhere herein.

As seen in FIG. 3, pressure generating device 4 also includes a power supply 34, a sensing circuit 36 and an analog-to-digital converter 38 operatively coupled to sensing circuit 36 and processing portion 28. Modem 36 is structured and configured to enable pressure support system 4 to communicate with central computer system 6 over a suitable network, such as the Internet. Pressure generating device 4 may also include a modem (wired or wireless) or similar device (not shown) structured and configured to enable pressure support system 4 to communicate with a remotely located computer system over a suitable network, such as the Internet. The function and purpose of each of the elements is described in more detail elsewhere herein.

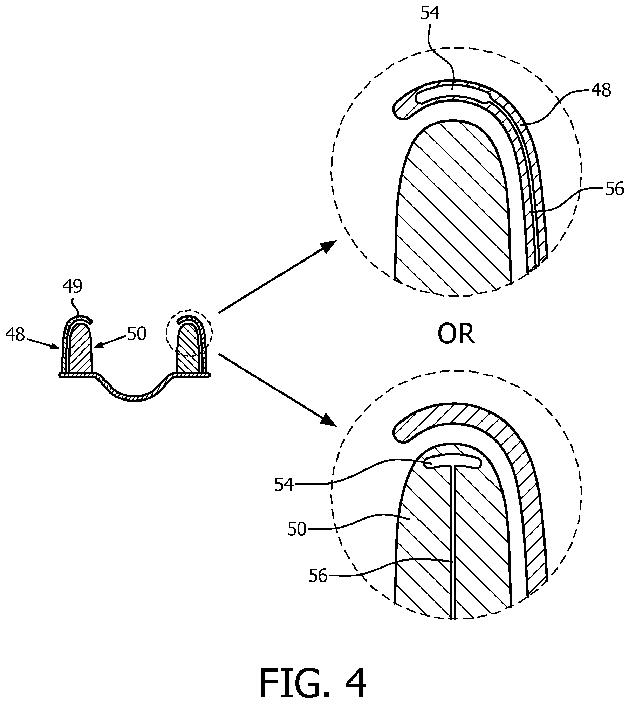

As shown schematically in FIG. 4 (which includes a cross-sectional view taken along lines A-A of FIG. 1), in the exemplary embodiment, cushion member 14 includes a sealing flap portion 48 defining a sealing surface 49 and a support portion 50 provided beneath sealing flap portion 48. Also in the exemplary embodiment, cushion member 14 comprises a unitary piece of soft, flexible, cushiony, elastomeric material, such as, without limitation, silicone or an appropriately soft thermoplastic elastomer, or any combination of such materials. It will be understood, however, that cushion member 14 does not need to be unitary within the scope of the present invention. Rather, cushion member 14, and the parts thereof, may be made of separate components (e.g., separate sealing flap portion 48 and support portion 50) that are coupled to one another by suitable means. In addition, as best seen in FIG. 1, the exemplary cushion member 14 has a generally triangular shape (i.e., the outline of sealing surface 49 is generally triangular in shape) including a bottom region 40, an apex region 42 located opposite bottom region 40, a first side region 44 and a second side region 46 located opposite first side region 44.

Furthermore, in the exemplary embodiment shown in FIGS. 1-3, cushion member 14 is provided with an electrode array 52 comprising a plurality of electrode elements 54 provided and spaced along and aligned with the outline of sealing surface 49. In particular, in the present illustrated exemplary embodiment, at any location along the outline of the sealing surface 49, there is exactly one electrode element 54 directly aligned therewith (as will be appreciated, some locations along the outline of the sealing surface 49 will have no electrode elements 54 directly aligned therewith). In addition, as demonstrated in alternative form in FIG. 4, each electrode element 54 may be provided within sealing flap portion 48 or within support portion 50 (in this respect, projected capacitance technology is advantageous as it does not require each electrode element 54 to actually touch the face in order to make the measurements). In further alternatives, each electrode element 54 may be provided at other locations on or in cushion member 14, such as, without limitation, on the top surface of sealing flap portion 48 or on the top surface of support portion 50.

Each electrode element 54 comprises a conductive member made of a suitable conductive material, such as, without limitation, a metal, a conductive polymer, a conductive liquid or a conductive silicone (a silicone material having conductive additives mixed therein). In addition, in the illustrated exemplary embodiment (which, as noted elsewhere herein, employs self capacitance technology), each electrode element 54 is individually electrically coupled to power supply 39 and sensing circuit 36 of pressure generating device 4 by way of a respective lead or leads 56 provided on or within cushion member 14 that are coupled to a wired connection 58 provided along elbow conduit 12 and delivery conduit 6. Cushion member 14 may be formed in this configuration using an appropriate molding and/or injection process.

Sensing circuit 36 of the present exemplary embodiment includes a number of electronic components that are configured and adapted to measure the current capacitance value (in analog form) of each electrode element 54. The analog capacitance value signals may then digitized by A/D 38 and provided to processing portion 28. As a result, in operation, when patient interface device 8 is donned by a user (e.g., for use in therapy), a resulting ΔC (i.e., the resulting change in capacitive as compared to some baseline value when patient interface device 8 is not being worn) can be calculated for each electrode element 54. As described herein, the magnitude of such ΔC measurements may be used to detect the degree of contact between the user's face and various regions of sealing surface 49 of cushion member 14 to identify locations of potential leak, over-tightening and/or optimum fit.

As noted elsewhere herein, in an alternative embodiment, it is also possible to implement patient interface device 8 using mutual capacitance technology. In such an alternative embodiment, each electrode element 52 does not need to be individually electrically coupled to power supply 39 and sensing circuit 36. Instead, suitable electrical connections are made to electrode elements 54 such that sensing circuit 36 is able to measure (in analog form) the change in mutual capacitance (ΔMC) associated with each of a number of associated neighboring electrode elements 54 in response to patient interface device 8 being donned by a user. As described herein, the magnitude of such ΔMC measurements may be used to detect the degree of contact between the user's face and various regions of sealing surface 49 of cushion member 14 to identify locations of potential leak, over-tightening and/or optimum fit.

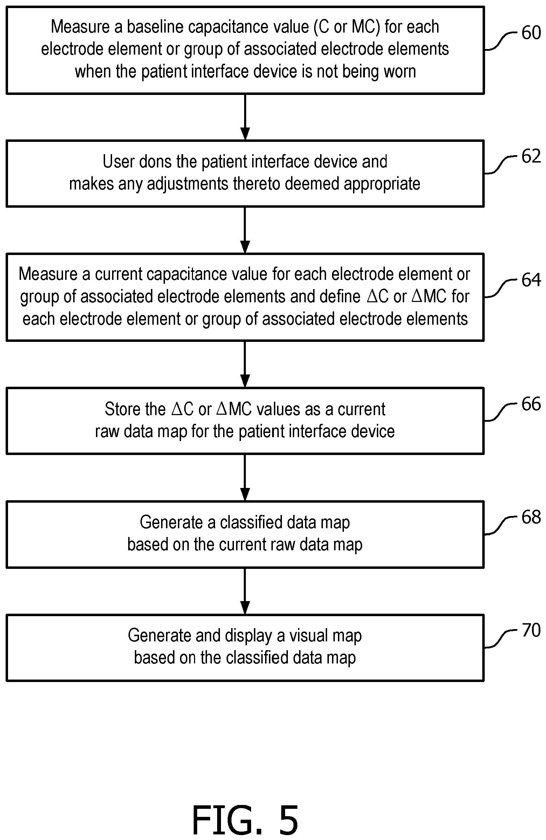

FIG. 5 is a flowchart which illustrates one exemplary embodiment of a method for detecting the degree of contact between the user's face and various regions of sealing surface 49 of cushion member 14 based on the measurements made using electrode array 52 (in either a self capacitance or mutual capacitance implementation as just described). As will be appreciated, in the exemplary embodiment, certain steps of the method of FIG. 5 will be implemented in one or more software routines executed by processing portion 28 of pressure generating device 4. In the exemplary embodiment, the method of FIG. 5 begins at step 60, wherein baseline capacitance values are measured for patient interface device 8 when it is not being worn. In the case of the self capacitance implementation, the baseline capacitance value is a capacitance (C) and it is measured for each electrode element 54 by sensing circuit 36 and provided to processing portion 28 as described herein. In the case of the mutual capacitance implementation, the baseline capacitance value is a mutual capacitance (MC) and it is measured for each group of associated electrode elements 54 (e.g., two neighboring electrode elements 54) by sensing circuit 36 and provided to processing portion 28 as described herein. In an alternative embodiment, step 60 may be omitted. In particular, in such an embodiment, the capacitive properties of the electrode elements 54 can be sufficiently characterized such that the baseline measurement is not required.

Next, at step 62, the user dons patient interface device 8 and makes any adjustments thereto deemed appropriate (e.g., headgear straps may be tightened and/or other fit adjustments may be made). Then, at step 64, current capacitance values (C or MC as appropriate depending on the implementation) are measured for patient interface device 8, and processing portion 28 determines either a ΔC for each electrode element 54 (in the case of the self capacitance implementation) or a ΔMC for each group of associated electrode elements 54 (in the case of the mutual capacitance implementation). At step 66, the calculated ΔC or ΔMC values are stored as a current raw data map for patient interface device 8.

As will be appreciated, in such a raw data map, each position along the outline of sealing surface 49 will have an associated ΔC or ΔMC value. Next, at step 68, in the present embodiment, each of the ΔC or ΔMC values is classified into categories according to a predetermined methodology as indicating one of (i) potential leak, (ii) optimum fit, and (iii) over-tightening in order to generate and store a classified data map. As will be appreciated, in such a classified data map, each position along the outline of sealing surface 49 will have an associated classified/categorized data item. A number of different exemplary predetermined classification methodologies that may be used in step 68 are described below. Next, at step 70, a visual map is generated and displayed based on the classified data map. A number of particular exemplary implementations of such a visual map that may be generated and displayed in step 70 are also described below. Furthermore, in addition to or instead of the generation and display of a visual map in step 70, a number of other actions may be taken based on the current raw data map and/or the classified data map. Examples of such other actions are described elsewhere herein.

In an alternative embodiment of the method of FIG. 5, the measurement sequence including steps 64 to 70 are triggered by a leak alarm from pressure generating device 4. Also, the estimated leak values from pressure generating device 4 (calculated as described elsewhere herein) can be used to convert the ΔC or ΔMC values for each electrode element 54 or group of associated electrode elements 54 into real leakage flow rates.

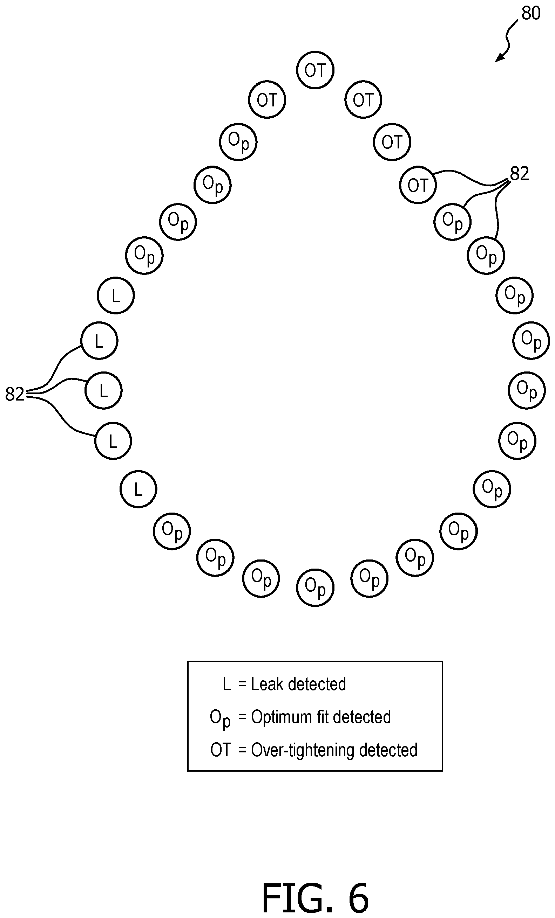

In one particular exemplary embodiment, the classified data map and corresponding visual map are generated as follows. First, each value in the raw data map is classified as follows: (i) ΔC or ΔMC ≤ low threshold value classified as indicating potential leak (L); (ii) low threshold value > ΔC or ΔMC < high threshold value classified as indicating optimum fit (Op); and (iii) ΔC or ΔMC ≥ high threshold value classified as indicating over-tightening (OT). Then, a visual map 80 as shown schematically in FIG. 6 is generated. As seen in FIG. 6, visual map 80 has a shape that matches the outline of sealing surface 49 of cushion member 14. Visual map 80 has a plurality of indicators 82 that are spaced along the outline of sealing surface 49, wherein each indicator 82 corresponds to and visually indicates the raw data (step 66) and resulting classification (step 68) that is associated with that position on the outline. As a result, a viewer (e.g., patient or caregiver), upon viewing visual map 80 as displayed on, for example, display 34 (FIG. 3), can readily see areas of potential leak, optimum fit and/or over-tightening so that appropriate adjustments can be made.

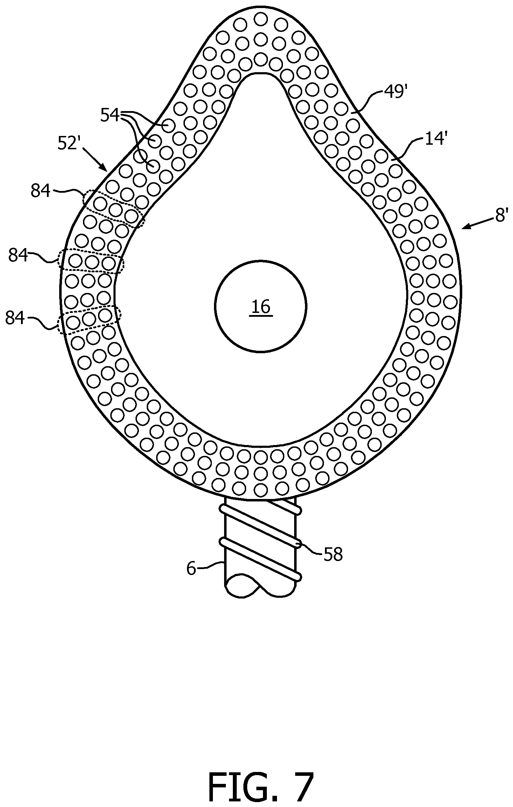

A number of alternative particular exemplary embodiments employ the alternative patient interface device 8' shown schematically in FIG. 7. Patient interface device 8' is similar to patient interface device 8, and like components are labeled with like reference numerals. However, as seen in FIG. 7, alternative cushion member 14' (similar to cushion member 14) has an alternative electrode array 52' that, at each of a plurality of locations along the outline of sealing surface 49', has a group 84 of two or more electrode elements 54 associated therewith.

In one embodiment employing the alternative patient interface device 8', during steps 64 and 66 of FIG. 5, a ΔC value is determined for each electrode element 54 in the group 84. Then, in step 68, the classified data map is generated by determining a classification (potential leak, optimum fit or over-tightening) for each group 84 that combines the individual ΔC values for the group 84. In one particular, non-limiting implementation, the classification for each group 84 is determined by first averaging the ΔC values for the group 84 and then classifying the average ΔC value using the low and high threshold values as described above to create the classified data map. That classified data map may then be used to generate a visual map 80 as shown in FIG. 6.

In another alterative embodiment employing the alternative patient interface device 8', during steps 64 and 66 of FIG. 5, a ΔMC value is determined for each group 84 using the electrode elements 54 in the group 84 (i.e., the electrode elements 54 in each group 84 are associated with one another for mutual capacitance determination). Then, in step 68, the classified data map is generated by determining a classification (potential leak, optimum fit or over-tightening) for each group 84 based on the ΔMC value for the group 84 and the low and high threshold values as described above. That classified data map may then be used to generate a visual map 80 as shown in FIG. 6.

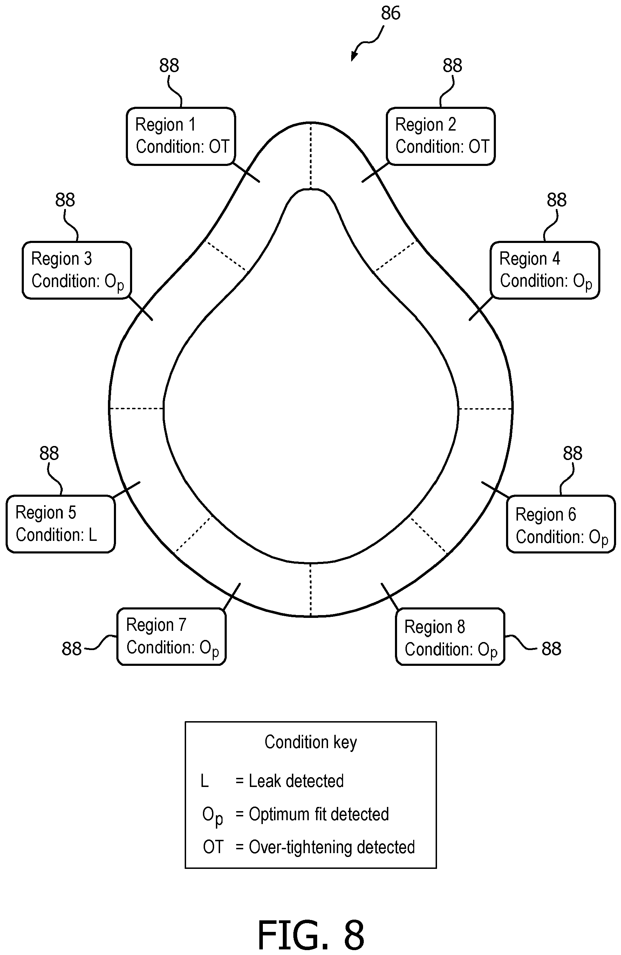

In still another alternative embodiment employing the alternative patient interface device 8', a visual map 86 as shown FIG. 8 is generated. In this embodiment, cushion member 14', and in particular sealing surface 49', is divided into a number of different regions. In the non-limiting, illustrated exemplary embodiment of FIG. 8, eight such regions (labeled "Region 1" through "Region 8") are provided and include the following: left apex (Region 1), right apex (Region 2), upper left side (Region 3), upper right side (Region 4), lower left side (Region 5), lower right side (Region 6), left bottom (Region 7) and right bottom (Region 8). A condition (potential leak, optimum fit, over-tightening) is determined for each region and displayed as shown in FIG. 8. More specifically, during steps 64 and 66 of FIG. 5, a ΔC or ΔMC value (depending on the particular implementation) is determined and stored for each electrode element 54 (self capacitance) or group of associated electrode elements 54 (mutual capacitance) in the electrode array 52'.

Then, in step 68, the classified data map is generated by first determining for each of the electrode elements 54 whether it has a ΔC or ΔMC that is equal to or greater than a contact threshold value (a level chosen that would indicate a certain degree of contact/touching). Next, for each region (1 through 8 in the illustrated embodiment), the number (n_{contact}) of electrode elements 54 therein that have a ΔC or ΔMC that is equal to or greater than the contact threshold value is determined. Then, each region (1 through 8 in the illustrated embodiment) is classified based on the n_{contact} value determined for that region as follows: (i) n_{contact} ≤ low contact number classified as indicating potential leak (L); (ii) low contact number > n_{contact} < high contact number classified as indicating optimum fit (Op); and (iii) n_{contact} ≥ high contact number classified as indicating over-tightening (OT). That classified data map may then be used to generate a visual map 86 as shown in FIG. 8 having an indicator 88 for each region.

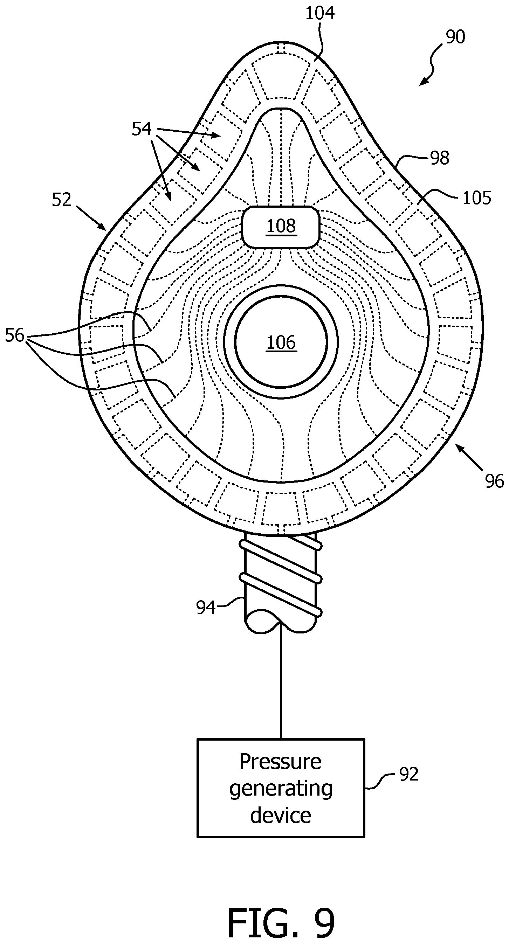

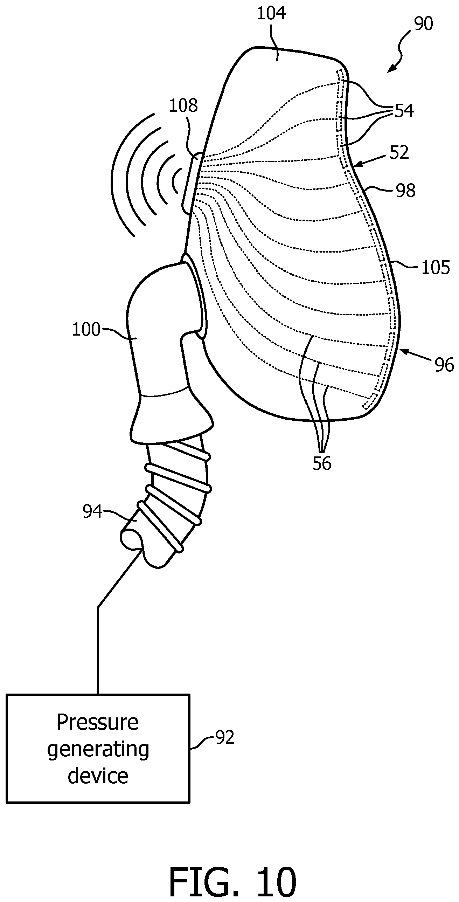

FIGS. 9 and 10 are front and side schematic views, respectively, of a system 90 adapted to provide a regimen of respiratory therapy to a patient according to a further alternative exemplary embodiment. System 90 may be employed to implement the various embodiments of the method of FIG. 5 to create a raw data map, a classified data map and/or a visual map as described elsewhere herein. For illustrative purposes, and as described in detail herein, in the exemplary embodiment of FIGS. 9 and 10, self capacitance technology is employed to enable the automated determination of potential leak, over-tightened and/or optimum fit regions of a patient interface device. It will be understood, however, that that is meant to be exemplary only and that other PCT implementations, such as mutual capacitance technology, may also be employed to implement the principles of the present invention.

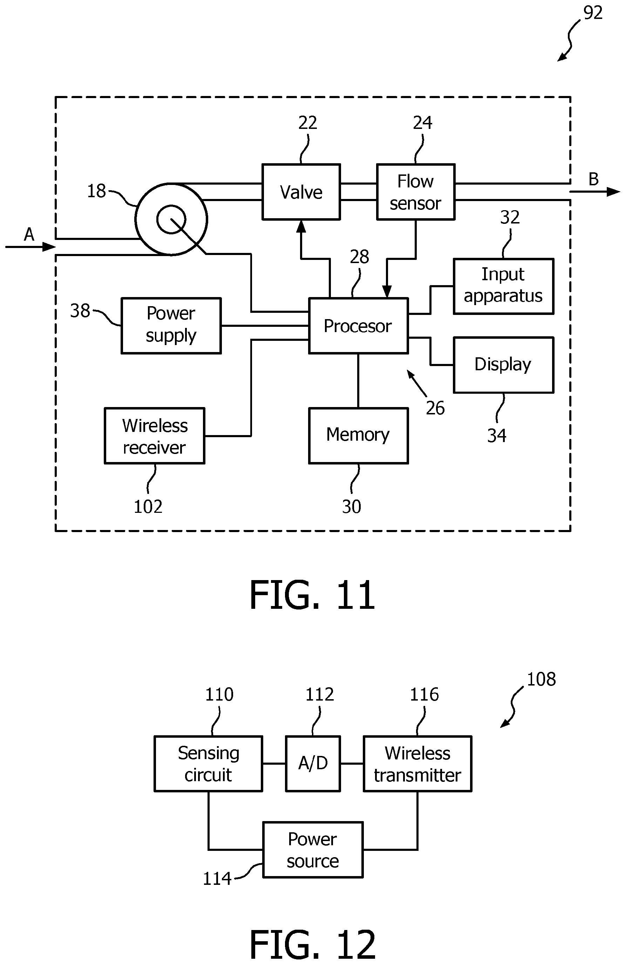

As seen in FIGS. 9 and 10, system 90 includes a pressure generating device 92, a delivery conduit 94, and a patient interface device 96 including a mask component 98 and an elbow conduit 100. Pressure generating device 92 is shown in FIG. 11 and is similar to pressure generating device 4 shown in FIG. 3 (like components are labeled with like reference numerals). Pressure generating device 98, however, does not include sensing circuit 36 and A/D 38, and instead includes wireless receiver 102, the function of which is described elsewhere herein.

In the illustrated embodiment, mask component 98 comprises a nasal mask structured to cover the nose of the patient. However, other types of mask components, such as, without limitation, a nasal/oral mask that covers the patient's nose and mouth, a nasal cushion that is positioned beneath and engages the patient's nose, or a full face mask that covers the patient's face, which facilitate the delivery of the flow of breathing gas to, and the removal of a flow of exhalation gas from, the airway of a patient may be used while remaining within the scope of the present invention. In the embodiment shown in FIGS. 9 and 10, mask component 98 includes a cushion member 104 having an opening 106 to which elbow conduit 100 is coupled to allow the flow of breathing gas from pressure generating device 92 to be communicated to an interior space defined by cushion member 104, and then, to the airway of a patient.

Furthermore, in the exemplary embodiment shown in FIGS. 9-10, cushion member 104 is provided with an electrode array 52 (or 52') as described elsewhere herein in various embodiments comprising a plurality of electrode elements 54 (as described elsewhere herein) provided and spaced along and aligned with the outline of sealing surface 105 of cushion member 104. In the exemplary embodiment of FIGS. 9 and 10, each electrode element 54 is electrically coupled to a local electronic device 108 by way of a respective lead or leads 56 provided in cushion member 104.

FIG. 12 is a schematic diagram of local electronic device 108 according to the exemplary embodiment. In the exemplary embodiment, local electronic device 108 is structured to measure and wirelessly transmit capacitance values to pressure generating 92 through wireless receiver 102.

As seen in FIG. 12, local electronic device 108 includes a sensing circuit 110, an analog-to-digital converter 112, a power source 114 and a wireless transmitter 116. Sensing circuit 110 of the present exemplary embodiment includes a number of electronic components that are configured and adapted to measure the current capacitance value (in analog form) of each electrode element 54. The analog capacitance value signals may then digitized by A/D 112 and provided to wireless transmitter 116. Wireless transmitter 116 is structured to transmit those values to wireless receiver 102 (FIG. 11) using any suitable wireless communications protocol, such as, without limitation, the Bluetooth® protocol. As a result, in operation, when patient interface device 96 is donned by a user (e.g., for use in therapy), a resulting ΔC (i.e., the resulting change in capacitive as compared to some baseline value when patient interface device 8 is not being worn) can be calculated for each electrode element 54 by processing portion 28 based on the capacitance information wirelessly received by pressure generating device 92 from local electronic device 108. As described herein, the magnitude of such ΔC measurements may be used to detect the degree of contact between the user's face and various regions of sealing surface 105 of cushion member 104 to identify locations of potential leak, over-tightening and/or optimum fit.

In an alternative embodiment, local electronic device 8 could also communicate with computing devices other than pressure generating 92 (e.g., without limitation, a stand-alone monitor, a personal computer, a tablet PC, etc.), with such other computing devices being configured and adapted to process the capacitance values in the manners described herein. In still another embodiment, all of the electronics (including the processing means for processing the capacitance values in the manners described herein) could be located in local electronic device 108.

As noted elsewhere herein, in an alternative embodiment, it is also possible to implement patient interface device 96 using mutual capacitance technology. In such an alternative embodiment, each electrode element 52 does not need to be individually electrically coupled to local electronic device 108. Instead, suitable electrical connections are made to electrode elements 54 such that sensing circuit 110 is able to measure (in analog form) the mutual capacitance associated with each of a number of associated neighboring electrode elements 54 in response to patient interface device 8 being donned by a user. As described herein, the magnitude of such measurements may be used to detect the degree of contact between the user's face and various regions of sealing surface 105 of cushion member 104 to identify locations of potential leak, over-tightening and/or optimum fit.

As stated elsewhere herein, in addition to or instead of the generation and display of a visual map in step 70 of FIG. 5, a number of other actions may be taken based on the current raw data map and/or the classified data map. For example, pressure generating device 4 or pressure generating device 92 may be adapted and programmed to generated intelligent alarms suggesting specific corrective action for the user based on the current raw data map and/or the classified data map. Such an alarm may be an audio signal and/or visual display instructing the user to take an action such as "tighten the top strap" or "loosen the bottom strap" to correct a detected condition such as a leak or over-tightening condition.

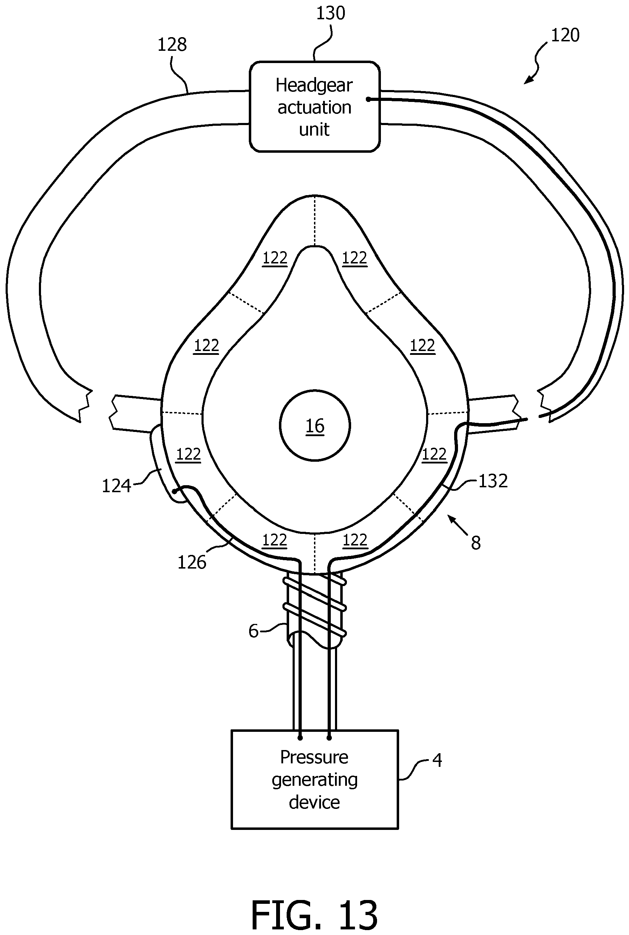

As another example, automatic adjustments may be made to a mask component based on the current raw data map and/or the classified data map. For instance, FIG. 13 shows an alternative system 120 that is similar to system 2 (like components being labeled with like reference numerals) described herein except that it further includes a number of components for making automatic adjustments in response to the generated raw data map and/or the classified data map. In particular, the patient interface device 8 including an electrode array as described herein in this embodiment includes one or both of the following additional features: (i) a mask component having a number of inflatable regions 122 fluidly coupled to a manifold 124 and pressure generating device 4 such that individual regions 122 can be selectively and separately automatically inflated through a line 126 based on the generated raw data map and/or the classified data map in order to automatically address and remedy conditions of concern, such as identified leak and/or over-tightened regions, and (ii) an automatically adjustable headgear component 128 having a headgear actuation unit 130 electrically coupled to pressure generating device 4 through a line 132 such that the size of headgear component 128 can be automatically adjusted based on the generated raw data map and/or the classified data map in order to automatically address and remedy conditions of concern, such as identified leak and/or over-tightened regions.

As still another example, the raw data map and/or classified data map information may be transmitted from pressure generating device 4 to the remote computer system of a caregiver or technician (using a modem to similar device as described elsewhere herein) to allow the caregiver or technician to remotely view the mask/face touch profile and troubleshoot without requiring a trip to the patient's home. The raw data map and/or classified data map information may also be transmitted from pressure generating device 4 to the remote computer system of product design teams to allow for assessment of actual use and improvements to future designs.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the invention has been described in detail for the purpose of illustration based on what is currently considered to be the most practical exemplary embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. A respiratory therapy system, comprising:
a patient interface device (8, 8', 96) capable of delivering breathing gas to a patient and having a contacting element (14, 14', 104) having a user contacting surface (49, 49', 105), wherein each of a plurality of locations along at least a portion of the contacting surface has a number of electrode elements (54) associated therewith;
a sensing circuit (36, 110) coupled to the electrode elements (54); and
a processing apparatus (26) in communication with the sensing circuit, **characterized in that** the sensing circuit and the processing apparatus are structured to: (i) determine a plurality of capacitance values using the electrode elements (54) when the patient interface device is donned by a user, wherein each of the capacitance values is associated with one of the locations, (ii) classify each of the capacitance values into one of a plurality of predetermined categories, and (iii) use the classified capacitance values to identify a number of predetermined fit conditions along the contacting element.

2. The respiratory therapy system according to claim 1, wherein the sensing circuit and the processing apparatus are part of a pressure generating device (4) structured to provide a flow of breathing gas to the patient interface device.

3. The respiratory therapy system according to claim 1, wherein the processing apparatus is part of a pressure generating device (4) structured to provide a flow of breathing gas to the patient interface device, and wherein the sensing circuit is provided as part of a local electronic device (108) coupled to the contacting element, the local electronic device being in wireless communication with the pressure generating device.

4. The respiratory therapy system according to claim 1, wherein the contacting element is a cushion member forming part of a mask component of the patient interface device.

5. The respiratory therapy system according to claim 1, the processing apparatus being structured to create and display on a display device a visual map (80, 86) for the patient interface device based on the classified capacitance values, the visual map comprising a plurality of indicators (82, 88), each of the indicators being associated with one of the locations and indicating one of the predetermined fit conditions.

6. The respiratory therapy system according to claim 1, the processing apparatus being structured to cause a component of the patient interface device to be automatically adjusted based on at least one of the identified number of predetermined fit conditions along the contacting element.

7. The respiratory therapy system according to claim 1, wherein the number of predetermined fit conditions includes a potential leak condition, an optimum fit condition and an over-tightened condition, wherein the plurality of predetermined categories includes a potential leak category, an optimum fit category and an over-tightened category, wherein each of the locations has a single one of the electrode elements associated therewith, wherein each of the capacitance values is associated with a respective one of the electrode elements, and wherein for each capacitance value the classifying comprises (i) classifying the capacitance value in the potential leak category if the capacitance value is less than or equal to a low threshold value, (ii) classifying the capacitance value in the optimum fit category if the capacitance value is greater than the low threshold value and less than a high threshold value, and (iii) classifying the capacitance value in the over-tightened category if the capacitance value is greater than or equal to the high threshold value.

8. The respiratory therapy system according to claim 1, wherein the number of predetermined fit conditions includes a potential leak condition, an optimum fit condition and an over-tightened condition, wherein the plurality of predetermined categories includes a potential leak category, an optimum fit category and an over-tightened category, wherein each of the locations has a group of the electrode elements associated therewith, wherein for each location the capacitance value is determined using the group of the electrode elements associated with the location, and wherein for each capacitance value the classifying comprises (i) classifying the capacitance value in the potential leak category if the capacitance value is less than or equal to a low threshold value, (ii) classifying the capacitance value in the optimum fit category if the capacitance value is greater than the low threshold value and less than a high threshold value, and (iii) classifying the capacitance value in the over-tightened category if the capacitance value is greater than or equal to the high threshold value.

9. The respiratory therapy system according to claim 8, wherein for each location the capacitance value is a change in mutual capacitance (ΔMC) determined using the group of the electrode elements associated with the location.

10. The respiratory therapy system according to claim 8, wherein for each location the capacitance value is an average change in capacitance (ΔC) determined using the group of the electrode elements associated with the location.

11. The respiratory therapy system according to claim 1, wherein the number of predetermined fit conditions includes a potential leak condition, an optimum fit condition and an over-tightened condition, wherein the contacting element has a plurality of regions, wherein for each capacitance value the classifying comprises determining whether the capacitance value is greater than or equal to a threshold value, and wherein the processing apparatus is structured to, for each region (i) determine a number of the capacitance values associated with that region that are greater than or equal to the threshold value and (ii) assign one of the predetermined fit conditions based on the determined number.

12. The respiratory therapy system according to claim 1, wherein each of the electrode elements is made from a conductive silicone.

13. A method of detecting a fit of a patient interface device (8, 8', 96) of a respiratory therapy system, the patient interface device (8, 8', 96) being capable of delivering breathing gas to a patient, the patient interface device having a contacting element (14, 14', 104) having a user contacting surface (49, 49', 105), wherein each of the capacitance values is associated with one of a plurality of locations along at least a portion of the contacting surface, wherein each of the locations has a number of electrode elements (54) associated therewith, the method being **characterized by**:
determining a plurality of capacitance values using the electrode elements (54), when the patient interface device is donned by a user;
classifying each of the capacitance values into one of a plurality of predetermined categories; and
using the classified capacitance values to identify a number of predetermined fit conditions along the contacting element.

14. The method according to claim 13, further comprising creating and displaying a visual map (80, 86) for the patient interface device based on the classified capacitance values, the visual map comprising a plurality of indicators (82, 88), each of the indicators being associated one of the locations and indicating one of the predetermined fit conditions.

15. The method according to claim 13, further comprising automatically adjusting a component of the patient interface device based on at least one of the identified number of predetermined fit conditions along the contacting element.

## Patentansprüche

1. Atemtherapiesystem, umfassend:
eine Patientenschnittstellenvorrichtung (8, 8', 96), die fähig ist, Atemgas zu einem Patienten zu liefern, und die ein Berührungselement (14, 14', 104) hat, das eine Benutzerberührungsoberfläche (49, 49', 105) hat, wobei jede einer Vielzahl von Stellen entlang mindestens eines Abschnitts der Berührungsoberfläche eine Anzahl von Elektrodenelementen (54), die damit assoziiert sind, hat;
eine Erfassungsschaltung (36, 110), die mit den Elektrodenelementen (54) gekoppelt ist, und
ein Verarbeitungsgerät (26) in Kommunikation mit der Erfassungsschaltung, **dadurch gekennzeichnet, dass** die Erfassungsschaltung und das Verarbeitungsgerät strukturiert sind, um: (i) eine Vielzahl von Kapazitätswerten unter Verwenden der Elektrodenelemente (54) zu bestimmen, wenn die Patientenschnittstellenvorrichtung von einem Benutzer angelegt wird, wobei jeder der Kapazitätswerte mit einer der Stellen assoziiert ist, (ii) jeden der Kapazitätswerte in eine einer Vielzahl vorbestimmter Kategorien einzustufen, und (iii) die eingestuften Kapazitätswerte zu verwenden, um eine Anzahl vorbestimmter Passbedingungen entlang des Berührungselements zu identifizieren.

2. Atemtherapiesystem nach Anspruch 1, wobei die Erfassungsschaltung und das Verarbeitungsgerät Teil einer Druckerzeugungsvorrichtung (4) sind, die strukturiert ist, um einen Fluss von Atemgas zu der Patientenschnittstellenvorrichtung bereitzustellen.

3. Atemtherapiesystem nach Anspruch 1, wobei das Verarbeitungsgerät Teil einer Druckerzeugungsvorrichtung (4) ist, die strukturiert ist, um einen Fluss von Atemgas zu der Patientenschnittstellenvorrichtung bereitzustellen, und wobei die Erfassungsschaltung als Teil einer lokalen elektronischen Vorrichtung (108), die mit dem Berührungselement gekoppelt ist, bereitgestellt ist, wobei die lokale elektronische Vorrichtung in drahtloser Kommunikation mit der Druckerzeugungsvorrichtung steht.

4. Atemtherapiesystem nach Anspruch 1, wobei das Berührungselement ein Kissenelement ist, das Teil eines Maskenbauteils der Patientenschnittstellenvorrichtung bildet.

5. Atemtherapiesystem nach Anspruch 1, wobei das Verarbeitungsgerät strukturiert ist, um auf einer Displayvorrichtung eine visuelle Darstellung (80, 86) für die Patientenschnittstellenvorrichtung basierend auf den klassifizierten Kapazitätswerten zu schaffen und anzuzeigen, und wobei die visuelle Darstellung eine Vielzahl von Indikatoren (82, 88) umfasst, wobei jeder der Indikatoren mit einer der Stellen assoziiert ist und einen der vorbestimmten Passzustände angibt.

6. Atemtherapiesystem nach Anspruch 1, wobei die Verarbeitungsvorrichtung strukturiert ist, um ein Bauteil der Patientenschnittstellenvorrichtung zu veranlassen, automatisch basierend auf mindestens einem der identifizierten Anzahl vorbestimmter Passzustände entlang Berührungselements eingestellt zu werden.

7. Atemtherapiesystem nach Anspruch 1, wobei die Anzahl vorbestimmter Passzustände einen potentiellen Leckzustand, einen optimalen Passzustand und einen übermäßig festgezogenen Zustand aufweist, wobei die Vielzahl vorbestimmter Kategorien eine potentielle Leckkategorie, eine optimale Passkategorie und eine Kategorie übermäßigen Festziehens aufweist, wobei mit jeder der Stellen ein einziges der Elektrodenelemente assoziiert ist, wobei jeder der Kapazitätswerte mit einem jeweiligen der Elektrodenelemente assoziiert ist, und wobei für jeden Kapazitätswert (i) die Einstufung das Klassifizieren des Kapazitätswerts in die potentielle Leckkategorie umfasst, falls der Kapazitätswert kleiner oder gleich einem unteren Schwellenwert ist, (ii) die Einstufung des Kapazitätswerts in die optimale Passkategorie umfasst, falls der Kapazitätswert größer ist als der untere Schwellenwert und kleiner als ein hoher Schwellenwert, und (iii) das Einstufen des Kapazitätswerts in die übermäßig festgezogene Kategorie, falls der Kapazitätswert größer oder gleich dem höheren Schwellenwert ist.

8. Atemtherapiesystem nach Anspruch 1, wobei die Anzahl vorbestimmter Passzustände einen potentiellen Leckzustand, einen optimalen Passzustand und einen Zustand des übermäßigen Festziehens aufweist, wobei die Vielzahl vorbestimmter Kategorien eine potentielle Leckkategorie, eine optimale Passkategorie und eine Kategorie des übermäßigen Festziehens aufweist, wobei mit jeder der Stellen eine Gruppe von Elektrodenelementen assoziiert ist, wobei für jede Stelle der Kapazitätswert unter Verwenden der Gruppe von Elektrodenelementen, die mit der Stelle assoziiert ist, bestimmt wird, und wobei für jeden Kapazitätswert das Einstufen (i) das Einstufen des Kapazitätswerts in die potentielle Leckkategorie umfasst, falls der Kapazitätswert kleiner oder gleich einem unteren Schwellenwert ist, (ii) das Einstufen des Kapazitätswerts in die optimale Passkategorie, falls der Kapazitätswert größer ist als der untere Schwellenwert und kleiner als ein hoher Schwellenwert ist, und (iii) das Einstufen des Kapazitätswerts in die Kategorie übermäßigen Festziehens, falls der Kapazitätswert größer oder gleich dem oberen Schwellenwert ist.

9. Atemtherapiesystem nach Anspruch 8, wobei für jede Stelle der Kapazitätswert eine Änderung der gegenseitigen Kapazitanz (ΔMC) ist, die unter Verwenden der Gruppe von Elektrodenelementen, die mit der Stelle assoziiert ist, bestimmt wird.

10. Atemtherapiesystem nach Anspruch 8, wobei für jede Stelle der Kapazitätswert eine mittlere Änderung der Kapazitanz (ΔC) ist, die unter Verwenden der Gruppe von Elektrodenelementen, die mit der Stelle assoziiert ist, bestimmt wird.

11. Atemtherapiesystem nach Anspruch 1, wobei die Anzahl vorbestimmter Passzustände einen potentiellen Leckzustand, einen optimalen Passzustand und einen übermäßig festgezogenen Zustand aufweist, wobei das Berührungselement eine Vielzahl von Bereichen hat, wobei für jeden Kapazitätswert das Einstufen das Bestimmen umfasst, ob der Kapazitätswert größer oder gleich einem Schwellenwert ist, und wobei das Verarbeitungsgerät strukturiert ist, um für jeden Bereich (i) eine Anzahl von Kapazitätswerten, die mit diesem Bereich assoziiert ist, zu bestimmen, die größer oder gleich dem Schwellenwert sind, und (ii) einem der vorbestimmten Passzustände basierend auf der bestimmten Anzahl zuzuweisen.

12. Atemtherapiesystem nach Anspruch 1, wobei jedes der Elektrodenelemente aus einem leitfähigen Silikon gefertigt ist.

13. Verfahren zum Erfassen des Passens einer Patientenschnittstellenvorrichtung (8, 8', 96) eines Atemtherapiesystems, wobei die Patientenschnittstellenvorrichtung (8, 8', 96) fähig ist, Atemgas zu einem Patienten zu liefern, wobei die Patientenschnittstellenvorrichtung ein Berührungselement (14, 14', 104) hat, das eine Benutzerberührungsoberfläche (49, 49', 105) hat, wobei jeder der Kapazitätswerte mit einer einer Vielzahl von Stellen entlang mindestens eines Abschnitts der Berührungsoberfläche assoziiert ist, wobei mit jeder der Stellen eine Anzahl von Elektrodenelementen (54) assoziiert ist, Verfahren **gekennzeichnet durch**:
Bestimmen einer Vielzahl von Kapazitätswerten unter Verwenden der Elektrodenelemente (54), wenn die Patientenschnittstellenvorrichtung von einem Benutzer angelegt wird;
Einstufen jedes der Kapazitätswerte in eine Vielzahl vorbestimmter Kategorien, und
Verwenden des klassifizierten Kapazitätswerts zum Identifizieren einer Anzahl vorbestimmter Passzustände entlang des Berührungselements.

14. Verfahren nach Anspruch 13, das ferner das Anlegen und Anzeigen einer visuellen Darstellung (80, 86) für die Patientenschnittstellenvorrichtung basierend auf den eingestuften Kapazitätswerten umfasst, wobei die visuelle Darstellung eine Vielzahl von Indikatoren (82, 88) umfasst, wobei jeder der Indikatoren mit einer der Stellen assoziiert ist und einen der vorbestimmten Passzustände angibt.

15. Verfahren nach Anspruch 13, das ferner das automatische Einstellen eines Bauteils der Patientenschnittstellenvorrichtung basierend auf mindestens einer der identifizierten Anzahl vorbestimmter Passzustände entlang des Berührungselements umfasst.

## Revendications

1. Système de thérapie respiratoire, comprenant :
un dispositif d'interface patient (8, 8', 96) capable de distribuer un gaz respiratoire à un patient et ayant un élément de mise en contact (14, 14', 104) ayant une surface de mise en contact avec l'utilisateur (49, 49', 105), dans lequel chacun d'une pluralité d'emplacements le long d'au moins une partie de la surface de mise en contact comporte un nombre d'éléments d'électrode (54) associés à ceux-ci ;
un circuit de détection (36, 110) couplé aux éléments d'électrode (54) ; et
un appareil de traitement (26) en communication avec le circuit de détection, **caractérisé en ce que** le circuit de détection et l'appareil de traitement sont structurés pour : (i) déterminer une pluralité de valeurs de capacité en utilisant les éléments d'électrode (54) quand le dispositif d'interface patient est revêtu par un utilisateur, dans lequel chacune des valeurs de capacité est associée à l'un des emplacements, (ii) classer chacune des valeurs de capacité dans l'une d'une pluralité de catégories prédéterminées, et (iii) utiliser les valeurs de capacité classées pour identifier un nombre de conditions d'ajustement prédéterminées le long de l'élément de mise en contact.

2. Système de thérapie respiratoire selon la revendication 1, dans lequel le circuit de détection et l'appareil de traitement font partie d'un dispositif de génération de pression (4) structuré pour apporter un écoulement de gaz respiratoire jusqu'au dispositif d'interface patient.

3. Système de thérapie respiratoire selon la revendication 1, dans lequel l'appareil de traitement fait partie d'un dispositif de génération de pression (4) structuré pour apporter un écoulement de gaz respiratoire jusqu'au dispositif d'interface patient, et dans lequel le circuit de détection est procuré comme partie d'un dispositif électronique local (108) couplé à l'élément de mise en contact, le dispositif électronique local étant en communication sans fil avec le dispositif de génération de pression.

4. Système de thérapie respiratoire selon la revendication 1, dans lequel l'élément de mise en contact est un élément coussin formant une partie d'un composant de masque du dispositif d'interface patient.

5. Système de thérapie respiratoire selon la revendication 1, l'appareil de traitement étant structuré pour créer et afficher sur un dispositif d'affichage une carte visuelle (80, 86) pour le dispositif d'interface patient sur la base des valeurs de capacité classées, la carte visuelle comprenant une pluralité d'indicateurs (82, 88), chacun des indicateurs étant associés à l'un des emplacements et indiquant l'une des conditions d'ajustement prédéterminées.

6. Système de thérapie respiratoire selon la revendication 1, l'appareil de traitement étant structuré pour amener un composant du dispositif d'interface patient à être automatiquement ajusté sur la base d'au moins une du nombre identifié de conditions d'ajustement prédéterminées le long de l'élément de mise en contact.

7. Système de thérapie respiratoire selon la revendication 1, dans lequel le nombre de conditions d'ajustement prédéterminées inclut une condition de fuite potentielle, une condition d'ajustement optimal et une condition trop serrée, dans lequel la pluralité de catégories prédéterminées inclut une catégorie fuite potentielle, une catégorie ajustement optimal et une catégorie trop serré, dans lequel chacun des emplacements possède un seul des éléments d'électrode associé à celui-ci, dans lequel chacune des valeurs de capacité est associée à un des éléments d'électrode respectif, et dans lequel, pour chaque valeur de capacité, le classement comprend (i) le classement de la valeur de capacité dans la catégorie fuite potentielle si la valeur de capacité est inférieure ou égale à une valeur de seuil faible, (ii) le classement de la valeur de capacité dans la catégorie ajustement optimal si la valeur de capacité est supérieure à la valeur de seuil faible et inférieure à une valeur de seuil élevé, et (iii) le classement de la valeur de capacité dans la catégorie trop serré si la valeur de capacité est supérieure ou égale à la valeur de seuil élevé.

8. Système de thérapie respiratoire selon la revendication 1, dans lequel le nombre de conditions d'ajustement prédéterminées inclut une condition de fuite potentielle, une condition d'ajustement optimal et une condition trop serrée, dans lequel la pluralité de catégories prédéterminées inclut une catégorie fuite potentielle, une catégorie ajustement optimal et une catégorie trop serré, dans lequel chacun des emplacements possède un groupe des éléments d'électrode associés à celui-ci, dans lequel, pour chaque emplacement, la valeur de capacité est déterminée en utilisant le groupe des éléments d'électrode associés à l'emplacement, et dans lequel, pour chaque valeur de capacité, le classement comprend (i) le classement de la valeur de capacité dans la catégorie fuite potentielle si la valeur de capacité est inférieure ou égale à une valeur de seuil faible, (ii) le classement de la valeur de capacité dans la catégorie ajustement optimal si la valeur de capacité est supérieure à la valeur de seuil faible et inférieure à une valeur de seuil élevé, et (iii) le classement de la valeur de capacité dans la catégorie trop serré si la valeur de capacité est supérieure ou égale à la valeur de seuil élevé.

9. Système de thérapie respiratoire selon la revendication 8, dans lequel, pour chaque emplacement, la valeur de capacité est un changement de capacité mutuelle (ΔMC) déterminé en utilisant le groupe des éléments d'électrode associés à l'emplacement.

10. Système de thérapie respiratoire selon la revendication 8, dans lequel, pour chaque emplacement, la valeur de capacité est un changement moyen de capacité (ΔC) déterminé en utilisant le groupe des éléments d'électrode associés à l'emplacement.

11. Système de thérapie respiratoire selon la revendication 1, dans lequel le nombre de conditions d'ajustement prédéterminées inclut une condition de fuite potentielle, une condition d'ajustement optimal et une condition trop serrée, dans lequel l'élément de mise en contact comporte une pluralité de régions, dans lequel, pour chaque valeur de capacité, le classement comprend le fait de déterminer si la valeur de capacité est supérieure ou égale à une valeur de seuil, et dans lequel l'appareil de traitement est structuré pour, chaque région, (i) déterminer un nombre des valeurs de capacité associées à cette région qui sont supérieure ou égales à la valeur de seuil et (ii) attribuer l'une des conditions d'ajustement prédéterminées sur la base du nombre déterminé.

12. Système de thérapie respiratoire selon la revendication 1, dans lequel chacun des éléments d'électrode se compose d'un silicone conducteur.

13. Procédé de détection d'un ajustement d'un dispositif d'interface patient (8, 8', 96) d'un système de thérapie respiratoire, le dispositif d'interface patient (8, 8', 96) étant capable de distribuer un gaz respiratoire à un patient, le dispositif d'interface patient ayant un élément de mise en contact (14, 14', 104) ayant une surface de mise en contact avec l'utilisateur (49, 49', 105), dans lequel chacune des valeurs de capacité est associée à l'un d'une pluralité d'emplacements le long d'au moins une partie de la surface de mise en contact, dans lequel chacun des emplacements comporte un nombre d'éléments d'électrode (54) associés à celui-ci, le procédé étant **caractérisé par** :
la détermination d'une pluralité de valeurs de capacité en utilisant les éléments d'électrode (54), quand le dispositif d'interface patient est revêtu par un utilisateur ;
le classement de chacune des valeurs de capacité dans l'une d'une pluralité de catégories prédéterminées ; et
l'utilisation des valeurs de capacité classées pour identifier un nombre de conditions d'ajustement prédéterminées le long de l'élément de mise en contact.

14. Procédé selon la revendication 13, comprenant en outre la création et l'affichage d'une carte visuelle (80, 86) pour le dispositif d'interface patient sur la base des valeurs de capacité classées, la carte visuelle comprenant une pluralité d'indicateurs (82, 88), chacun des indicateurs étant associé à l'un des emplacements et indiquant l'une des conditions d'ajustement prédéterminées.

15. Procédé selon la revendication 13, comprenant en outre l'ajustement automatique d'un composant du dispositif d'interface patient sur la base d'au moins une du nombre identifié de conditions d'ajustement prédéterminées le long de l'élément de mise en contact.
